# EUROPEAN PATENT SPECIFICATION

(11) **EP 4 133 283 B1**
(45) Date of publication and mention of the grant of the patent: **15.01.2025**
(21) Application number: 21717800.3
(22) Date of filing: 06.04.2021
(51) Int. Cl.: G01N 33/68, G01N 33/84, G01N 33/94

(54) **SELENOPROTEIN P AS MARKER FOR SELENIUM INTOXICATION**
SELENOPROTEIN P ALS MARKER FÜR SELENVERGIFTUNG
SÉLÉNOPROTÉINE P EN TANT QUE MARQUEUR D'INTOXICATION AU SÉLÉNIUM

(30) Priority: 07.04.2020 DE 102020002289
(43) Date of publication of application: 15.02.2023
(73) Proprietor: Selenomed GmbH, 10965 Berlin (DE)
(72) Inventor: SEEMANN, Petra, 10965 Berlin (DE)
(74) Representative: Gruber, Daniel
(86) International application number: PCT/EP2021/058841
(87) International publication number: WO 2021/204745

(56) References cited:
- VINCETI MARCO ET AL: "Environmental Selenium and Human Health: an Update", CURRENT ENVIRONMENTAL HEALTH REPORTS, vol. 5, no. 4, 1 December 2018 (2018-12-01), DE, pages 464 - 485, XP055802370, ISSN: 2196-5412, Retrieved from the Internet <URL:http://link.springer.com/content/pdf/10.1007/s40572-018-0213-0.pdf> DOI: 10.1007/s40572-018-0213-0
- BURK RAYMOND F. ET AL: "Effects of Chemical Form of Selenium on Plasma Biomarkers in a High-Dose Human Supplementation Trial", CANCER EPIDEMIOLOGY, BIOMARKERS & PREVENTION, vol. 15, no. 4, 1 April 2006 (2006-04-01), pages 804 - 810, XP055802373, ISSN: 1055-9965, Retrieved from the Internet <URL:https://cebp.aacrjournals.org/content/15/4/804.full-text.pdf> DOI: 10.1158/1055-9965.EPI-05-0950
- XIA YIMING ET AL: "Optimization of selenoprotein P and other plasma selenium biomarkers for the assessment of the selenium nutritional requirement: a placebo-controlled, double-blind study of selenomethionine supplementation in selenium-deficient Chinese subjects", AMERICAN JOURNAL OF CLINICAL NUTRITION, vol. 92, no. 3, 1 September 2010 (2010-09-01), pages 525 - 531, XP055802379, ISSN: 0002-9165, Retrieved from the Internet <URL:https://www.ncbi.nlm.nih.gov/pmc/articles/PMC2921536/pdf/ajcn9230525.pdf> DOI: 10.3945/ajcn.2010.29642
- BORNHORST JULIA ET AL: "The crux of inept biomarkers for risks and benefits of trace elements", TRAC TRENDS IN ANALYTICAL CHEMISTRY, vol. 104, 15 November 2017 (2017-11-15), pages 183 - 190, XP085410102, ISSN: 0165-9936, DOI: 10.1016/J.TRAC.2017.11.007
- BRODIN OLA ET AL: "Selenoprotein P as Biomarker of Selenium Status in Clinical Trials with Therapeutic Dosages of Selenite", NUTRIENTS, vol. 12, no. 4, 12 April 2020 (2020-04-12), pages 1067 - 8, XP055802472, Retrieved from the Internet <URL:https://www.ncbi.nlm.nih.gov/pmc/articles/PMC7230801/pdf/nutrients-12-01067.pdf> DOI: 10.3390/nu12041067

## Description

Instant invention refers to methods for evaluating the grade of Se intoxication in a subject by determining a measured value of SePP and correlating said measured value with a Selenium intoxication classification category; and one or more health status parameter of said subject; and/or one or more disease specific parameter of said subject and the use of the methods in the analysis and monitoring of Selenium poisoning, therapeutical Se supplementation and animal feed.

Selenoprotein P (abbreviations Seppl , SeP, SELP, SePP, SELENOP, hereinafter "SePP") is a plasma selenoprotein known to serve as marker of selenium (Se) deficiency (Burk and Hill 2015). Its serum or plasma concentration reaches a saturation level that is used as indicator of a sufficiently high, i.e., replete and health-supporting Se intake (Hurst, Armah et al. 2010, Combs, Jackson et al. 2012). Furthermore, the determination of the SePP status has become an important method for the assessment of the nutritional Se requirement (Ashton, Hooper et al. 2009, Xia, Hill et al. 2010, Kipp, Strohm et al. 2015).

Structurally, human SePP is a protein containing 381 amino acid residues (SwissProt number P49908) of which ten are predicted to be Sec residues at positions 59, 300, 318, 330, 345, 352, 367, 369, 376 and 378. Its secreted form (after cleavage of the signal sequence) contains 362 amino acid residues and may contain post-translational modifications, which can include phosphorylation and multiple sites of glycosylation (Burk and Hill 2005, Saito 2020). Moreover, several variants including fragments containing the N- or C-terminal part of SePP have been described (Saito, Sato et al. 2004, Burk and Hill 2005, Ballihaut, Kilpatrick et al. 2012).

Circulating concentrations of SePP have been shown to be saturable, i.e., SePP is supposed to reach a maximal concentration in the circulation of 5-7 mg/l under normal conditions (Hill, Xia et al. 1996, Hurst, Armah et al. 2010). It is therefore a suitable biomarker for Se deficiency and for the determination of Se requirement. Se supplementation studies have indicated that SePP serum or plasma concentration is an easily accessible marker of human Se nutritional status, whereas quantification of the trace element concentration of Se in the blood requires more efforts due to the need of physico-chemical/spectroscopic techniques (e.g., atom absorption spectrometry, X-ray fluorescence or mass spectrometry) (Persson-Moschos, Alfthan et al. 1998, Xia, Hill et al. 2005, Burk, Norsworthy et al. 2006, Hurst, Armah et al. 2010, Xia, Hill et al. 2010, Rayman, Searle et al. 2014, Kahaly, Riedl et al. 2017).

Populations and subjects with sub-optimal expression levels with circulating concentrations of less than 5 mg/l SePP are considered and classified as Se-deficient, i.e., in need of supplemental Se administration. Once, the Se serum concentration is replete, i.e., in the range of at least 120-130 µg Se/l, circulating SePP becomes saturated and it has been reported that SePP concentration does not increase above 5-7 mg/l, even upon further Se supplementation (Burk, Norsworthy et al. 2006, Hurst, Armah et al. 2010, Xia, Hill et al. 2010). The respective subject with 5-7 mg/l of SePP is considered as Selenium-replete. It was believed that higher intake levels of Se are not affecting the circulating SePP concentrations any further, and that SePP concentrations above 5-7 mg/l may represent a rare status, resulting from a specific genotype, disease or other individual reasons, but are not caused by high Se intake alone. Very high SePP concentrations have been described in relation to obesity (Chen, Liu et al. 2017) or pulmonary arterial hypertension (Kikuchi, Satoh et al. 2018), but not in response to supplementation with Se-containing substances.

Consequently, a protein or peptide biomarker for intakes greater than the tolerable Upper intake Level (UL) of 400 µg Se/day was outside from consideration in the art (Combs 2015, Vinceti, Filippini et al. 2018). No peptide or protein candidate has been suggested as a suitable marker for monitoring such very high Se intakes. The only diagnostic method available were physico-chemical/spectroscopic techniques quantifying the total serum, plasma or blood Se concentrations (Vinceti, Filippini et al. 2018). Using these techniques, cases of Se toxicity have been analyzed and serum Se concentrations above the reference values have been reported (e.g., paradise nut paradox) causing eventually hair loss as well as finger nail and toe nail weakness and loss (Senthilkumaran, Balamurugan et al. 2012, Morris and Crane 2013).

Several SePP quantification methods are known, with antibody-based immunoassays as the most wide-spread and frequently used (Perssonmoschos, Huang et al. 1995, Hill, Xia et al. 1996, Hollenbach, Morgenthaler et al. 2008, Tanaka, Saito et al. 2016, Hybsier, Schulz et al. 2017). Commercially available SePP ELISA kits are, e.g., Human Selenoprotein P (SELENOP) ELISA kit, Product Code: STE from selenOmed GmbH, Berlin, Germany; Chicken Selenoprotein P (SEPP1) ELISA Kit, Cat. No. AE56582CH from Wuhan Abebio science Co., Ltd., Wuhan City, China; Rabbit Selenoprotein P (SEPP1) ELISA Kit, Cat. No. AE56581RB from Wuhan Abebio science Co., Ltd. and others.

While SePP is particularly known as a suitable marker for monitoring the Se metabolism in deficient subjects, i.e., the dynamic progression of the Se status after Se intake of a subject towards a replete Se status, it was completely unexpected that it is possible to observe and interpret SePP concentrations higher than 7 mg/l in humans. Surprisingly, it was found that there is a dynamic increase in the concentration of SePP above the assumed threshold value of SePP saturation, i.e., above 7 mg/l, e.g., upon consecutive daily intake of Se amounts above the UL (i.e., more than 400 µg Se/day). These findings indicate that the described maximal saturation level of SePP in human blood as taught in the prior art does not represent a threshold value of maximum SePP concentration, but is the plateau of a polynomial curve, whereby a further increase of SePP concentration can be observed in response to continued Se supplementation by dosages considered as toxic.

These findings unexpectedly allow to use SePP as a marker for the detection and/or monitoring of an elevated Se status or Se intoxication status of a subject in response to Se intake, especially in case of intakes exceeding the recommended safe amounts (in humans the UL(Se) of 400 µg/day).

A number of diseases and disease risks are caused by Se deficiency, which can be treated or prevented by administration of supplemental selenium. Known examples that can be prevented by Se supplementation are Keshan disease or Kashin-Beck disease that constituted endemic diseases in Se poor areas of Asia (Yu, Han et al. 2016, Zhou, Wang et al. 2018). In addition, a growing number of diseases become targeted by the adjuvant therapeutic administration of high-dosed Se (Se therapy), i.e., far above the UL(Se) to a subject, in the preclinical (Sonkusre 2019) or clinical setting (Brodin, Eksborg et al. 2015). Therefore, there is the need in a cheap, fast, and reliable determination and monitoring of high Se supplementation effects in a subject, who is object of a Se therapy in order to monitor the Se effects and prevent Se intoxication of the subject during the performance of the therapy.

Due to the increased awareness that Se is likewise an important factor of the health status of animals, Se supplementation is of increasing interest in animal breeding and animal feed, to improve milk quality (Smith, Hogan et al. 1997), meat quality (Juniper, Phipps et al. 2008) or female (Kommisrud, Osteras et al. 2005) and male fertility (Ebeid 2009), respectively. Therefore, there is also the need in a cheap, fast, and reliable determination or monitoring of high Se supplementation effects in an animal individual to avoid Se intoxication of the animal during supplemental Se application or accidental Se intoxication in animal breeding and in animal feed. Thus, instant invention allows for the first time the provision of a method for the monitoring and prevention of potential Se intoxication in animal and human individuals by the use of SePP as biomarker for toxic Se intake.

It is one of the underlying problems of instant invention to provide a cheap, fast, and reliable determination of high level SePP in the blood of a subject, thereby allowing the control and monitoring of the appropriate amount of Se in supplementation therapy, which is to be administrated to an individual in need thereof, thereby allowing to safeguard the avoidance of Se intoxication, especially, to avoid chronical-progressive Se intoxication, i.e., selenosis.

It is another underlying problem of the present invention to allow the diagnostic use of a protein/peptide marker for monitoring Se supplementation, especially therapeutic or nutritional supplementation (with Se dosages exceeding the recommended safe dosages, i.e., exceeding the UL(Se) of 400 µg/d in humans), by correlating a protein/peptide concentration of SePP, i.e., SELENOP or its orthologues in animals in the blood of an individual with health- or disease-specific parameters of said individual, above the threshold value of greater than 7, especially 8, 9, or 10 mg/l, respectively.

The invention in its general sense is defined by independent claim 1.

The method of the invention is an in-vitro method. At least the step of determining a measured value of SePP in a sample of said subject in the methods of the invention is performed in-vitro.

In other embodiments the methods of instant invention may optionally apply the correlation of more than one measured value with one or more health status and/or disease specific parameters of the subject for diagnostic, risk stratification, prognostic, classifying and/or monitoring purposes during Se supplementation therapy.

Another object of instant invention according to claim 1 is a method for the monitoring of the SePP blood concentration during Se supplementation of a subject in need thereof, wherein the measured value corresponds to a SePP concentration in the blood of said individual of above 7 mg/l, preferably above 8 mg/l, more preferred above 9 mg/l, and most preferred above 10 mg/l in humans, and the corresponding concentrations in animals.

Disclosed but not claimed is a method for the assessment of the required Se amount for a subject during Se supplementation therapy of a subject in need thereof, comprising the steps of a) administrating a defined daily amount of supplemental Se to said individual and b) determining the measured value of SePP in a sample of said subject, respectively, the use of the method of the invention in such a method.

In one embodiment of the method for the assessment of the required Se amount for a subject during Se supplementation therapy of a subject in need thereof, comprises a step c) wherein the amount of supplemental Se is set to zero, if the measured value of SePP corresponds to the level of Se intoxication classification category D, which is greater than 15 mg/l, preferably greater than 10 mg/l.

Disclosed but not claimed is a method of monitoring the Se supplementation in a subject in need thereof, wherein the measured value corresponds to a SePP concentration in the blood of said individual of above 7 mg/l, preferably above 8 mg/l, more preferred above 9 mg/l, and most preferred above 10 mg/l.

The term "Se supplementation" or "Se therapy" according to instant invention preferably means an amount of supplemental Se exceeding the recommended safe intake amount, as defined by the 'tolerable Upper intake Limit' (UL(Se)) of Selenium, especially for therapeutic or preventive purposes, and may include animal feed supplementation. The term "UL(Se)" according to instant invention means preferably the recommended "tolerable Upper intake Level" of Se for the respective species. According to the governmental food safety authorities 400 µg/Se per day is currently defined as the UL(Se) ("tolerable Upper intake Level", cf. https://ods.od.nih.gov/factsheets/Selenium-HealthProfessional/) for humans. Accordingly, the UL(Se) for each respective animal species or race is an equivalent thereof based on the veterinary knowledge of the skilled person. In this respect, it is known that SePP concentrations and the amount of Se per SePP molecule show species-specific differences in relation to the Se requirements of different vertebrates (Penglase, Hamre et al. 2015). E.g., the UL(Se) value for animals is most commonly expressed in the food content of Selenium, which is, e.g., for cattle 300µg/kg dry matter.

The term "subject" as used herein refers to a living human subject. The subject may be healthy or diseased if not stated otherwise. The term "subject" according to other embodiments of instant invention means preferably an individual suffering from a disease selected from sepsis, SIRS (systemic inflammatory response syndrome), infection, cardio-vascular disease (CVD) including atherosclerosis and heart failure, cancer, trauma, diabetes, or a severe illness treated on the intensive care unit (ICU) or suffering from poisoning.

The term "Se intoxication" according to instant invention means preferably a value of blood SePP concentration of greater than 7 mg/l for humans, preferably greater than 8 mg/l, and especially greater than 10 mg/l. For the purposes of an intoxication classification according to instant invention the following threshold scheme is applied for SePP concentrations in blood, plasma or serum (see figure):
Category A: Se deficiency corresponding to less than 5 mg/l;
Category B: Optimum Se status, no intoxication corresponding to 5-7 mg/l;
Category C: Increasing risk of Se intoxication corresponding to 7-10 mg/l;
Category D: Se intoxication corresponding to greater than 10 mg/l.

The skilled person is free to use a more refined classification scheme in accordance with physiological (health status) and/or pathophysiological (disease related or disease-specific) parameters of the subject under investigation.

Threshold levels can be obtained for instance from a Kaplan-Meier plot analysis or other more refined statistical methods (Huang 2018), where the occurrence of a disease or the probability of a serious condition and/or death is correlated with the e.g. percentiles of the respective markers in the subject or population, as e.g. shown for mortality risk in response to Se supplementation (Rayman, Winther et al. 2018). Notably, different human populations seem to adapt their set-point for Se status according to Se availability (White, Romagne et al. 2015), and may thus present with slightly different values for optimal Se intake range and SePP concentration and thresholds for UL(Se).

Accordingly, the skilled person may re-scale and adapt the said category values A to D with respect to needs of certain human populations and animal species or animal races, respectively, according to suitable medical, veterinary, animal feed, and animal breeding parameters, e.g., infection rate, cytokine concentration, fur condition, behavior, and/or the weight of a subject. It is also well within the knowledge of the skilled person that the values of SePP concentration of categories A to D in a particular animal species may differ from the numbers outlined for a particular human population.

The data of human populations indicate that an optimal Se status is achieved in the phase where SePP concentrations show a plateau with respect to Se intake, i.e., in the range of 5-7 mg/l SePP in European and US subjects, respectively. It can be assumed that a more precise plateau value can be identified in different human populations or animal species, respectively.

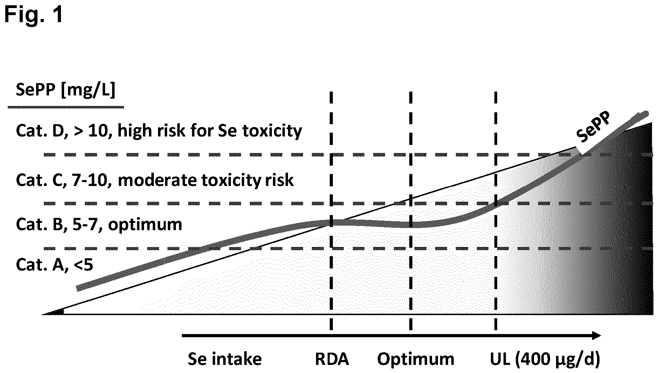

The optimal Se intake levels can be determined based on the intake amount that causes SePP concentrations in the plateau of the curve shown in Figure 1, i.e., that lead to SePP concentrations of category B.

The thresholds to category A and category C correspond to intakes causing SePP concentrations below or above the turning point of the curve shown in Fig. 1, which can be calculated by a polynomial curve fitting (e.g., by using a cubic parable as basis) or any other mathematical model known in the art. The turning point of the curve can be determined by constructing the first or second derivative and determining of the intercept of zero (root), as known in the art. The lower threshold defining category B is defined by a Se intake causing 50%, 60%, 70%, or 80% of the intake needed for optimal SePP concentration equal to the turning point of the curve shown schematically in Fig. 1.

Likewise, the upper threshold defining category B (the population- or animal-specific UL(Se)) can be defined by a Se intake causing 200%, 140%, 130%, or 120% of the intake needed for SePP concentrations equal to the turning point of the curve shown schematically in Fig. 1.

Above this threshold, the discrimination between categories C and D can likewise be deduced as multiples of the Se intake needed to achieving optimal SePP concentration, i.e., SePP concentration that corresponds to the turning point of the polynomial description of the curve shown in Fig. 1.

In a first embodiment of instant invention the term "SePP" means human SePP (SELENOP), e.g., encoded by GenBank database entry CAA77836.2, respectively, according to SwissProt database number P49908.

In a second embodiment of instant invention the term "SePP" means orthologues of SELENOP matching the respective species of the subject under investigation (Penglase, Hamre et al. 2015).

In an alternative embodiment of instant invention the term "SePP" means a specific fragment of human SePP, respectively, any orthologue thereof, which is suitable for quantification and as a marker of SePP as known in the art. Suitable fragments for the quantification of SePP are, e.g., disclosed in WO2019081504A1, cf. Seq ID No.'s 3 to 15 thereof.

The term "health status" according to instant invention preferably means the condition of the subject defined by one or more physiological (in case of a healthy subject) or pathophysiological parameters of the disease the subject is suffering from, e.g., cancer biomarkers in case of malignancy; cytokines in case of infection, sepsis or SIRS, blood oxygen saturation in respiratory disease, glomerular filtration rate in case of renal disease, blood pressure, heart ejection volume and/or heart rate in case of CVD, blood glucose or Hb1Ac in case of diabetes, or any other parameter defining organ dysfunction, which is well known by the skilled person in the art.

In another embodiment of the method of the present invention the measured value is obtained by means of an immunoassay and/or mass spectrometry. The term "immunoassay" according to instant invention preferably means any validated assay, which is based on antigen-antibody interaction and is, preferably an ELISA.

Another object of the present invention is the use of the instant method in the monitoring of therapeutic or nutritional Se supplementation.

The term "therapeutic or nutritional Se supplementation" according to instant invention preferably means the Se intake by a subject, which exceeds the tolerable upper intake limit (UL(Se)) according to the knowledge of the skilled person in the art. Another embodiment of the invention is the use of the methods of the invention for the quantification of the required individual Se amount and dosage regime during Se supplementation therapy in a subject.

Another embodiment of the invention is the use of the methods of the invention in the analysis and monitoring of Se poisoning.

Another embodiment of the invention is the use of the methods of the invention in the treatment monitoring of a subject suffering from a disease selected from sepsis, infection, (poly)trauma or SIRS.

Still another embodiment of the invention is the use of the methods of the invention in the treatment monitoring of a subject suffering from a viral infection, including viral hepatitis. acquired immune deficiency syndrome (AIDS), influenza A and corona virus, especially COVID-19, respectively.

Yet another embodiment of the invention is the use of the methods of the invention in the treatment monitoring of a subject suffering from cardio-vascular disease (CVD) or cancer.

Yet another embodiment of the invention is the use of the methods of the invention in the treatment monitoring of a subject suffering from a severe illness requiring treatment on the intensive care unit (ICU).

Yet another embodiment of the invention is the use of the methods of the invention in the treatment monitoring of a subject suffering from poisoning, i.e., for the monitoring in the treatment of a subject.

Disclosed but not claimed is the use of the methods of the invention in the treatment of a subject by administrating one or more compounds comprising pharmaceutically acceptable Se, or application of Se antidotes, i.e., substances intended to lower Se absorption and metabolism, e.g. therapeutic antibodies targeting SePP-receptors or SePP.

The term "pharmaceutically acceptable Se" according to instant invention preferably means selenite, selenate, selenomethionine (L-selenomethionine), selenocysteine and derivatives thereof, which is known as a pharmaceutically acceptable Se source in the art. The "pharmaceutically acceptable Se" corresponds to the Se, which is supposed for supplementation, respectively, administration to a subject.

The term "therapeutic Se supplementation" according to instant invention means a daily dosage of Se above the UL(Se). Supplementation with Se may be optionally applied in combination with vitamins (e.g. vitamin E, vitamin C, vitamin A) and/or mineral nutrients (e.g. iodine, fluoride, zinc) and/or co-factors (e.g. coenzyme Q10) and/or other specific drugs or combinations of medication.

The term "sample" according to instant invention means blood, serum, and plasma.

The term "measured value" according to instant invention means preferably any detectable signal representing the amount or concentration of SePP or fragments thereof, which fragments are known in the art as indicator molecules suitable for the quantification of SePP, preferably in an immunoassay.

In one specific embodiment SePP is measured by means of an immunoassay using an antibody or an antibody fragment, which binds to SePP and/or fragments thereof.

### FIGURES

Figure 1 illustrates the relation of SePP concentration in the blood of a subject with the categories of Se intoxication

SePP concentration in relation to Se intake and toxicity. The recommended daily amount ('RDA' of 55 µg/day for adult human subjects) denotes the intake that meets the nutrient requirements of the majority of subjects in a population. Different studies have indicated that the optimum is reached when SePP is maximally expressed ("saturated"). Detectable SePP concentrations do not reach a maximum plateau level. The turning point of a polynomial curve is achieved by optimal Se intake and serves to define different categories of Se intoxication levels in a subject as defined.

### REFERENCES:

Ashton, K., L. et al. (2009). American J of Clinical Nutrition 89(6): 2025s-2039s.
Ballihaut, G., et al. (2012). Metallomics 4(6): 533-538.
Brodin, O., et al. (2015). Nutrients 7(6): 4978-4994.
Burk, R. F. and K. E. Hill (2005). Annu Rev Nutr 25: 215-235.
Burk, R. F. and K. E. Hill (2015). Annu Rev Nutr 35: 109-134.
Burk, R. F., et al. (2006). Cancer Epidemiol Biomarkers Prev 15(4): 804-810.
Chen, M. X. et al. (2017). Obesity Research & Clinical Practice 11(2): 227-232.
Combs, G. F., Jr. (2015). Nutrients 7(4): 2209-2236.
Combs, G. F. et al. (2012). Br J Nutr 107(10): 1514-1525.
Hill, K. E. et al. (1996). J Nutr 126(1): 138-145.
Hollenbach, B. et al. 2008. J Trace Elem Med Biol 22(1): 24-32.
Huang, B. (2018). Pharmaceutical Statistics 17(1): 49-60.
Hurst, R. et al. (2010). Am J Clin Nutr 91(4): 923-931.
Hybsier, S. et al. (2017). Redox biology 11: 403-414.
Juniper, D. T. et al. (2008). J Anim Sci 86(11): 3100-3109.
Kahaly, G. J. et al. (2017). J of Clin Endocrinology & Metabolism 102(11): 4333-41.
Kikuchi, N. et al. (2018). Circulation 138(6): 600-623.
Kipp, A. P. et al. (2015). J Trace Elem Med Biol 32: 195-199.
Kommisrud, E. et al. (2005). Acta Vet Scand 46(4): 229-240.
Mehdi, Y. and I. Dufrasne (2016). Molecules 21(4): 545.
Mita, Y. et al. (2017). Nat Commun 8(1): 1658.
Penglase, S. et al. (2015). PeerJ 3: e1244.
Persson-Moschos, M. et al. (1998). Eur J Clin Nutr 52(5): 363-367.
Perssonmoschos, M. et al. (1995). Analyst 120(3): 833-836.
Raisbeck, M. F. (2000). Vet Clin North Am Food Anim Pract 16(3): 465-480.
Rayman, M. P. et al. (2014). Br J Nutr 112(1): 99-111.
Rayman, M. P. et al. (2018). Free Radical Biology and Medicine 127: 46-54.
Saito, Y. (2020). Journal of Clinical Biochemistry and Nutrition 66(1): 1-7.
Saito, Y. et al. (2018). Biological & Pharmaceutical Bulletin 41(5): 828-833.
Saito, Y. et al. (2004). Biochemical J. 381: 841-846.
Senthilkumaran, S. et al. (2012). Int J Trichology 4(4): 283-284.
Smith, K. L. et al. (1997). J Anim Sci 75(6): 1659-1665.
Sonkusre, P. (2019). Front Oncol 9: 1541.
Tanaka, M. et al. (2016). J Clin Lab Anal 30(2): 114-122.
Vinceti, M. et al. (2018). Curr Environ Health Rep 5(4): 464-485.
White, L. et al. (2015). Mol Biol Evol 32(6): 1507-1518.
Xia, Y. M.et al. (2005). American J. of Clinical Nutrition 81(4): 829-834.
Xia, Y. M. et al. (2010). American J. of Clinical Nutrition 92(3): 525-531.
Yu, F. F. et al. (2016). Biol Trace Elem Res 170(1): 25-32.
Zhou, H. et al. (2018). Biol Trace Elem Res 186(1): 98-105.

### EXAMPLES

### Example 1: Assay description

The selenOtest ELISA is a chromogenic enzyme-linked immunosorbent assay, for the quantitative determination of human SePP in serum samples. It uses two different SePP specific monoclonal antibodies for the antigen capture and detection steps, essentially as described (Hybsier, Schulz et al. 2017). The SePP concentrations of the calibrators and controls have been validated against serial dilutions of NIST SRM 1950 Standard Reference Material (National Institute of Standards & Technology, Gaithersburg, Maryland, U.S.A.). The lower limit of quantification (LLOQ) was determined at a SePP concentration of 11.6 ng/l, and the upper limit of quantification (UL(Se)OQ) at 538.4 µg/l, thereby defining the working range in diluted serum samples at SePP concentrations between 11.6 and 538.4 µg/l. The intersection at 20% CV defines the limit of detection (LOD) and was reached at a SePP concentration of 6.7 µg/l, i.e., around 500-fold below average serum SePP concentrations of well-supplied human subjects. The signals were linear on dilution within the working range of the assay, and SePP was stable in serum for 24h at room temperature. Further details of the assay parameters are published (Hybsier, Schulz et al. 2017), and the assay has been proven as a most reliable product in comparison to other competing products (Saito, Misu et al. 2018).

### Example 2: Two thousand µg daily Selenium Study

Healthy adult subjects were taking 2,000 µg of Se in the form of sodium selenite (obtained as OTC nutritional supplement from Biogena Selenit 200 Kapseln - Der Selen-Booster mit 200 µg Selen in Form von Natriumselenit) in the morning for five consecutive days. SePP was determined by a Point-of-Care Assay, developed by selenOmed GmbH, Berlin, Germany. Results were verified by control measurements using selenOtest ELISA, which is designed for the detection of SELENOP and obtainable from selenOmed GmbH, Berlin, Germany. The ELISA was performed according to the manufacturer's instructions.

**Table 1. Effect of 2,000 µg Se/day as Selenite-Intake over 5 consecutive days**

| | **SELENOP [mg/l]** | **SELENOP [mg/l]** |
|---|---|---|
| **Day 1** | 4,1 | 3,7 |
| **Day 2** | 4,5 | 4,4 |
| **Day 3** | 7,6 | 7,1 |
| **Day 4** | 9,3 | 9,0 |
| **Day 5** | 12,2 | 11,1 |
| **Day 6*** | 8,8 | 11,0 |
| **Day 7*** | 7,9 | 7,5 |
| **Day 14*** * **washout** | 7,3 | 6,9 |

Person 1, male, 53y Person 2, female, 43y

At baseline, SePP of the healthy volunteers was in category A, i.e., below the optimal Se status. Already three days after supplemental intake of 2,000 µg Se/day, the category B of optimal Se range was surpassed. Unexpectedly, concentrations of SePP in blood exceeded a value of 10 mg/l in both healthy probands 5 days after supplementation start. Further supplemental Se intake was stopped after 5 days.

### Example 3: Correlation with Health Status

Intake of supplemental Se (2,000 µg/day) in the form of sodium selenite as described in Example 2 led to irregular heartbeat, sleep disturbance and a general sense of feeling unwell (self-reported). Symptoms ceased with declining SePP concentrations in both probands.

### Example 4: Importance in Animals

A characterization of the Se intake causing Se toxicity (UL(Se)) in animals can be performed on the basis of the current invention. Like in human populations, an optimal Se status in animals is achieved when SePP concentrations show a plateau with respect to the amount of Se intake, which depends e.g. in farm animals on the amount of Se in kg of dry matter of feed. In a respective feeding study in animals, a similar plateau of SePP in response to increasing Se intake will be observed. As an example, it is known that the nutritional requirements in cattle are estimated at 100 µg Se/kg dry matter (beef cattle) or 300 µg Se/kg dry matter (dairy cows or hyper muscular cattle races like Belgian Blue) (Mehdi and Dufrasne 2016). The maximum Se content of standard in animal foods is set at 300 µg/kg and 500 µg/kg dry matter in the U.S.A. and in Europe, respectively. In cattle, Se supplementation can be achieved via the drinking water, mineral salts, Se-enriched yeast preparations or Se can be injected, implanted as slow releasing preparation or given as bolus via the feed. Providing Se in amounts above 500 µg Se/kg dry matter will cause increased risk of toxicity (category C in Fig. 1) and increase SePP concentrations above the aforementioned plateau phase. Further Se supply will cause Se toxicity according to category D. This is observed when e.g. cattle (groups of n=10) is supplemented with 800 µg Se/kg dry matter, 2 mg Se/kg dry matter, 5 mg Se/kg dry matter, 7.5 mg Se/kg dry matter, and 10 mg Se/kg dry matter, respectively.

It is known from similar studies that signs of selenium toxicity start to appear with fodder containing 5 to 8 mg Se/kg dry matter, where chronic selenosis ("alkali disease") or acute selenosis ("blind staggers") are observed (Raisbeck 2000).

The thresholds from category B to category C, i.e., towards Se toxicity, can be determined in such experiments with the method taught by the present invention, i.e., with help of measuring SePP as biomarker of Se toxicity. The SePP concentrations determined in the groups of animals exposed to the Se supply by the fodder in the range of 0.1 to 10 mg Se/kg dry matter can be plotted as a polynomial curve, and modeled by polynomial curve fitting using e.g. a cubic parabel as basis or by another mathematical process known to experts in the art. The turning point of the curve can be determined by constructing the first or second derivative and determining of the intercept of zero (root), as known in the art. The Se intake needed for optimal SePP concentration will be equal to the turning point of the curve shown schematically in Fig. 1. The upper threshold defining category B (animal species-specific UL(Se)) can be defined by a Se intake causing 2-, 1.4-, 1.3-, or 1.2-times of the intake needed for SePP concentrations equal to the turning point. Similarly, the threshold between category C (moderate risk for Se toxicity) and D (high risk for Se toxicity) can then be defined by 2.5-, 3.0-, 4.0- or 5.0-times of the intake needed for SePP concentrations equal to the turning point.

A detailed determination of the UL(Se) for animals can be performed on the basis of the current invention. The methods of the invention can be used for the fast detection of Selenium intoxication in animals, thereby avoiding overdosage of Selenium supplementation.

## Claims

1. An in-vitro method for evaluating the grade of Se intoxication in a human subject exposed to Se intake exceeding the tolerable upper intake level (UL(Se)) of Selenium, which is 400 µg/day comprising
a) determining a measured value of selenoprotein P (SePP) in a blood, serum or plasma sample obtained from said subject;
b) correlating said measured value determined according to step a) with a Se intoxication classification category,
wherein the measured value of SePP is above a threshold, which is greater than 7.0 mg/l.

2. The method according to claim 1, wherein said correlation comprises a further correlation of more than one measured value with one or more health status and/or one or more disease specific parameter of said subject for diagnosis, risk stratification, prognosis, classifying and/or monitoring disease specific parameters of said subject.

3. The method according to one or more of claims 1 to 2, which is an immunoassay, preferably an ELISA.

4. The method according to one or more of claims 1 to 3, which includes mass spectrometry, fluorescence or absorption spectrometry.

## Patentansprüche

1. In-vitro Verfahren zur Evaluierung des Se-Vergiftungsgrades bei einem Menschen, der einer Se-Aufnahme ausgesetzt war, die den tolerierbaren oberen Aufnahmewert (UL(Se)) von 400 µg/Tag Selen überschreitet, umfassend:
a) Bestimmen eines Messwertes von Selenoprotein P (SePP) in einer Blut-, Serum oder Plasmaprobe besagter Person;
b) Korrelieren des gemäß Schritt a) bestimmten Messwertes mit einer Se-Intoxikationsklassifizierungskategorie, wobei der Messwert von SePP über einem Schwellenwert liegt, der größer als 7,0 mg/l ist.

2. Verfahren nach Anspruch 1, wobei die Korrelation eine weitere Korrelation von mehr als einem Messwert mit einem oder mehreren Gesundheitszuständen und/oder einem oder mehreren krankheitsspezifischem Parameter besagter Person zur Diagnose, Risikostratifizierung, Prognose, Klassifizierung und/oder Überwachung krankheitsspezifischer Parameter besagter Person umfasst.

3. Verfahren nach einem oder mehreren der Ansprüche 1 bis 2, der ein Immunoassay ist, vorzugsweise ein ELISA.

4. Verfahren nach einem oder mehreren der Ansprüche 1 bis 3, das Massenspektrometrie, Fluoreszenz- oder Absorptionsspektrometrie umfasst.

## Revendications

1. Méthode in vitro destinée à évaluer le degré d'intoxication au Se chez un sujet humain exposé à un apport de Se dépassant le taux d'apport supérieur (UL(Se)) tolérable du sélénium, qui est de 400 µg/jour, comprenant les étapes consistant à
a) déterminer une valeur mesurée de sélénoprotéine P (SePP) dans un échantillon de sang, de sérum ou de plasma obtenu auprès dudit sujet;
b) corréler ladite valeur mesurée déterminée conformément à l'étape a) à une catégorie de classification d'intoxication au Se,
dans laquelle la valeur mesurée de SePP est au-dessus d'un seuil, qui est supérieur à 7,0 mg/l.

2. Méthode selon la revendication 1, dans laquelle ladite corrélation comprend une corrélation supplémentaire de plus d'une valeur mesurée avec un ou plusieurs statuts de santé et/ou un ou plusieurs paramètres spécifiques d'une maladie dudit sujet pour diagnostic, stratification des risques, pronostic, classification et/ou surveillance de paramètres spécifiques d'une maladie dudit sujet.

3. Méthode selon une ou plusieurs des revendications 1 à 2, qui est un dosage immunologique, préférentiellement un ELISA.

4. Méthode selon une ou plusieurs des revendications 1 à 3, qui comprend une spectrométrie de masse, une spectrométrie par fluorescence ou une spectrométrie d'absorption.
